**Europäisches Patentamt**

**European Patent Office**

**Office européen des brevets**

(19)

(11) Publication number : **0 373 503 B1**

(12) **EUROPEAN PATENT SPECIFICATION**

(45) Date of publication of patent specification :
09.09.92 Bulletin 92/37

(51) Int. Cl.⁵ : **C02F 3/28, B01F 7/24, C05F 9/02**

(21) Application number : **89122553.4**

(22) Date of filing : **07.12.89**

(54) **Agitator-mixer for biogas systems.**

(30) Priority : **15.12.88 IT 3076388 U**

(43) Date of publication of application :
20.06.90 Bulletin 90/25

(45) Publication of the grant of the patent :
09.09.92 Bulletin 92/37

(84) Designated Contracting States :
**AT BE CH DE ES FR GB GR LI LU NL SE**

(56) References cited :
**FR-A- 1 074 817**
**FR-A- 2 532 301**
**GB-A- 2 138 795**
**LU-A- 56 186**

(73) Proprietor : **Faccia, Tiziano**
**Via Padova 102**
**I-35026 Conselve (Padova) (IT)**

(72) Inventor : **Faccia, Tiziano**
**Via Padova 102**
**I-35026 Conselve (Padova) (IT)**

(74) Representative : **Modiano, Guido et al**
**MODIANO, JOSIF, PISANTY & STAUB**
**Modiano & Associati Via Meravigli, 16**
**I-20123 Milano (IT)**

EP 0 373 503 B1

## Description

The present invention relates to an agitator-mixer for biogas systems.

It is known that solid urban waste, in particular organic waste, has a significant energy content.

If one furthermore takes into account the fact that enormous amounts of solid urban waste are currently produced every day, one can see why the constant search for new energy sources has sought to use the energy content of waste, which would consequently also lead to considerable advantages from an ecological point of view.

In particular, the use of the energy content of waste is currently aimed at collecting the biogases, including mainly methane, which are produced thereby by the decomposition, which normally occurs at an extremely slow rate in landfills, with the introduction of malodorous and dangerous exhalations into the atmosphere.

French Patent Application FR-A-2 532 301 is disclosing a digester for liquid organic waste in which mixing is carried out by an archimedean spiral sheathed in a cylindrical tube.

Further archimedean spiral mixing elements are disclosed in the documents LU-A-56 186 and FR-A-1 074 817 which describe respectively a malting apparatus for grain and a mixer for blending various kinds of grains like coffee beans. Said mixing elements disclosed by both such documents are also sheathed.

The document GB-A-2 138 817 discloses an auger blade, substantially cylindrically shaped and operated manually at a low speed of rotation.

The aim of the present invention is to provide a machine which can mix and agitate waste, so as to accelerate its transformation into compost for the production of biogas to be aspirated for energy recover purposes.

A consequent primary object is to provide a machine which effectively achieves the constant movement of all the raw matter fed to a biogas system.

Another object is to provide a machine the use whereof allows to reduce the volume of the solid urban waste currently produced.

Not least object is to provide a machine which can be easily obtained starting from commonly available elements and materials.

This aim, these objects and others which will become apparent hereinafter are achieved by an agitator-mixer for biogas systems, as defined in claim 1.

The characteristics and advantages of the invention will become apparent from the detailed description of an embodiment of the invention, illustrated only by way of non-limitative example in the accompanying drawings, wherein the only figure is a schematic sectional view of an agitator-mixer according to the invention.

With reference to the above figure, an agitator-mixer for biogas systems is generally indicated by the reference numeral 1 and comprises a preferably cylindrical tank 2 arranged with a vertical symmetry axis.

Said tank 2, which is fixed on the ground, can be conveniently made of concrete, masonry or advantageously of metallic material.

According to the invention, said tank 2 axially contains a mixing element comprising a rotating scroll 3 which is advantageously provided with a variable pitch.

Said scroll 3 is conveniently constituted by a band 4 of sheet metal welded to form a spiral along a rotating shaft 5.

Bearings are arranged at the ends of said shaft 5 and rotatably couple it to the bottom 6 of the tank 2 on one side and to the center of an upwardly arranged diametrical crosspiece 7 at the opposite end.

In particular the width of the metallic band gradually decreases from the bottom upward.

It should furthermore be noted that the rotating shaft 5 has, proximate to the bottom 6, a substantially frustumcone shaped portion 8 adapted to facilitate the movement of the mixed material toward the periphery of the tank.

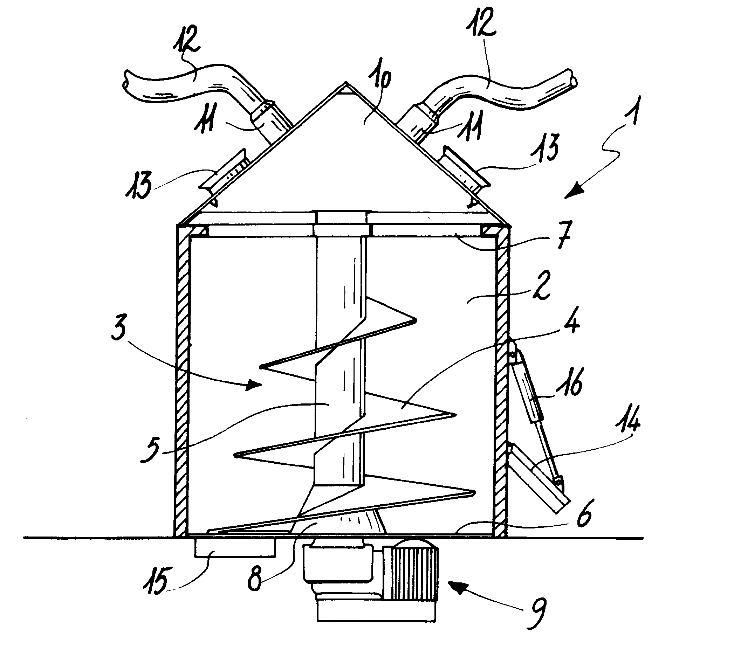

As can be seen in figure 1, the rotating scroll 3 is moved by a transmission unit 9 with an appropriate drive element, the entire assembly being arranged below the bottom 6.

Still according to the invention, a substantially conical hood 10, preferably made of metallic material, is arranged above said tank 2 and is coupled thereon with a gas-tight seal.

Gas intake pipes 12 are connected, by means of couplings 11, to the upper part of said hood 10.

It should be furthermore noted that loading doors 13 are provided on the hood 10 and unloading doors 14,15 are provided on the tank 2 respectively on the side wall and on the bottom thereof.

The loading and the unloading doors are gas-tight and can be moved for example by sliding or, as illustrated in the figure, by articulation about a pivoting point, said articulation being obtained by means of a piston 16.

From the above the operation of the agitator-mixer according to the invention is evident and can be summarized as follows.

The waste is introduced in the tank, through the loading doors 13, after partially removing the coarse inert materials and after possibly being subjected to shredding or trituration.

The scroll 3 has a continuous rotary motion which agitates and mixes the material contained therein, obtaining a compost the fermentation whereof releases a series of gases, the greatest amount whereof is generally constituted by methane.

Said gases, generally termed biogases, are collected in the hood 10 and sent to a possible utilization system through the pipes 12.

The material can be loaded at intervals of various duration, taking into account the fact that each charge requires at least one week of permanence inside the tank 2 in order to ferment completely.

When the fermentation reactions have ended, the compost is then unloaded outside through the doors 13 or 14.

It should be noted that the scroll 3, by virtue of its rotary motion, causes the product, which is at the center of the tank, to rise upward until such product reaches the top and then, after a side movement due to centrifugal force, falls by gravity along the walls.

In this manner a rise/fall motion is triggered which affects all the material contained in the tank 2, which is thus agitated and mixed uniformly.

From the above, it is clear that the aim and objects of the invention are therefore brilliantly achieved, an agitator-mixer having been devised which is studied for the continuous motion of the raw material required by a biogas system.

Said raw material may be constituted by organic waste but also by any other suitable material.

The agitator-mixer is constructively very simple and can be manufactured at low cost.

In practice, the materials employed and the dimensions may be any according to the requirements.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. An agitator-mixer for biogas systems comprising a tank (2) for mixing organic materials or the like, accommodating at least one mixing element (3) including a vertical-axis rotating scroll, said tank being upwardly provided with a biogas collecting hood (10), characterized in that suction tubes (12) are connected to said hood (10) and doors (13-15) are provided for loading and unloading said materials, said scroll (3) with its edge arranged on an ideal surface having a substantially conical shape, raising and centrifugating said materials towards the wall of said tank (2).

2. An agitator-mixer according to claim 1, characterized in that said scroll (3) is essentially constituted by a band (4) made of sheet metal which extends in a spiral along a rotating shaft (5) rotatably coupled to said tank (2).

3. An agitator-mixer according to claims 1 or 2, characterized in that said scroll (3) has a variable pitch.

4. An agitator-mixer according to any of the preceding claims, characterized in that said shaft (5) which supports said scroll (3) has a frustum-cone shaped lower part (8) and is actuated by a transmission (9), with drive unit, arranged below.

5. An agitator-mixer according to any of the preceding claims, characterized in that said hood (10) is fixed onto said tank (2) with a gas-tight coupling.

6. An agitator-mixer according to one or more of the preceding claims, characterized in that said loading doors (13) are arranged on said hood (10) and are gas-tight.

7. An agitator-mixer according to one or more of the preceding claims, characterized in that said unloading doors (14,15) are arranged on the side walls of said tank (2) and/or on its bottom (6) and are gas-tight.

## Revendications

1. Un agitateur-mélangeur pour des systèmes à biogaz, comprenant une cuve (2) prévue pour le mélange de matières organiques ou de matières semblables, contenant au moins un élément mélangeur (3) qui comporte une hélice tournante à axe vertical, cette cuve comportant à sa partie supérieure une hotte de collecte de biogaz (10), caractérisé en ce que des tubes d'aspiration (12) sont reliés à la hotte (10) et en ce que des portes (13-15) sont établies pour le chargement et le déchargement des matières précitées, l'hélice (3), dont le bord se trouve sur une surface idéale ayant une forme pratiquement conique, faisant monter et centrifugeant les matières précitées vers la paroi de la cuve (2).

2. Un agitateur-mélangeur selon la revendication 1, caractérisé en ce que l'hélice (3) est fondamentalement constituée par une bande (4) en tôle, qui s'étend en hélice le long d'un arbre tournant (5) entraîné en rotation dans la cuve.

3. Un agitateur-mélangeur selon la revendications 1 ou 2, caractérisé en ce que l'hélice (3) a un pas variable.

4. Un agitateur-mélangeur selon l'une quelconque des revendications précédentes, caractérisé en ce que l'arbre (5) qui supporte l'hélice (3) présente une partie inférieure tronconique (8) et est actionné par une transmission (9), avec un dispositif d'entraînement se trouvant au-dessous.

5. Un agitateur-mélangeur selon l'une quelconque des revendications précédentes, caractérisé en ce que la hotte (10) est fixée sur la cuve (2) avec un raccord étanche.

6. Un agitateur-mélangeur selon une ou plusieurs des revendicateurs précédentes, caractérisé en ce que les portes de chargement (13) se trouvent sur la hotte (10) et sont étanches.

7. Un agitateur-mélangeur selon une ou plusieurs des revendications précédentes, caractérisé en ce que les portes de déchargement (14, 15) se trouvent sur les parois latérales de la cuve (2) et/ou sur son fond (6) et sont étanches.

**Patentansprüche**

1. Rührwerk-Mischer für Biogassysteme, mit einem Tank (2) zum Mischen organischer Stoffe oder dergleichen, der zumindest ein Mischelement (3) mit einer vertikalachsig drehenden Schnecke aufweist und oben mit einer Biogas-Sammelhaube (10) versehen ist, **dadurch gekennzeichnet,** daß Saugleitungen (12) an die Sammelhaube (10) angeschlossen und Türen (13 - 15) zum Befüllen und Entleeren der genannten Stoffe vorgesehen sind, wobei die mit ihrem Rand auf einer ideellen Oberfläche angenähert konischer Form angeordnete Schnecke (3) die genannten Stoffe hochfördert und gegen die Wandung des Tankes (2) zentrifugiert.

2. Rührwerk-Mischer nach Anspruch 1, **dadurch gekennzeichnet,** daß die Schnecke (3) im wesentlichen aus einem Metallband (4) besteht, das sich in einer Spirale entlang einer Welle (5) erstreckt, die drehbar im Tank (2) gelagert ist.

3. Rührwerk-Mischer nach Anspruch 1 oder 2, **dadurch gekennzeichnet,** daß die Schnecke (3) eine sich ändernde Ganghöhe aufweist.

4. Rührwerk-Mischer nach Anspruch 1, 2 oder 3, **dadurch gekennzeichnet,** daß die die Schnecke (3) tragende Welle (5) einen kegelstumpfförmigen unteren beschnitt (8) aufweist und von einer eine Antriebseinheit aufweisenden, unterhalb angeordneten Transmission (9) beaufschlagt ist.

5. Rührwerk-Mischer nach einem der vorhergehenden Ansprüche, **dadurch gekennzeichnet,** daß die Sammelhaube (10) auf dem Tank (2) über eine gasdichte Verbindung festgelegt ist.

6. Rührwerk-Mischer nach einem oder mehreren der vorhergehenden Ansprüche, **dadurch gekennzeichnet,** daß die Befülltüren (13) in der Sammelhaube (10) angeordnet und gasdicht ausgebildet sind.

7. Rührwerk-Mischer nach einem oder mehreren der vorhergehenden Ansprüche, dadurch gekennzeichnet, daß die Entleerungstüren (14, 15) in den Seitenwandungen und/oder im Boden (6) des Tankes (2) angeordnet und gasdicht ausgebildet sind.